(19) Europäisches Patentamt / European Patent Office / Office européen des brevets

(11) **EP 2 955 501 B1**

(12) **EUROPEAN PATENT SPECIFICATION**

(45) Date of publication and mention
of the grant of the patent:
**06.06.2018 Bulletin 2018/23**

(21) Application number: **13878549.8**

(22) Date of filing: **03.04.2013**

(51) Int Cl.:
*G01N 33/49* (2006.01)     *A61B 5/053* (2006.01)

(86) International application number:
**PCT/CN2013/073740**

(87) International publication number:
**WO 2014/146312 (25.09.2014 Gazette 2014/39)**

(54) **SYSTEM FOR ERYTHROCYTE HEMATOCRIT MEASUREMENT AND OPERATION CONTROL METHOD**

SYSTEM ZUR ERYTHROZYTENHÄMATOKRITMESSUNG UND BETRIEBSSTEUERUNGSVERFAHREN

SYSTÈME DE MESURE D'HÉMATOCRITE D'ÉRYTHROCYTE ET PROCÉDÉ DE COMMANDE DE FONCTIONNEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.03.2013 CN 201310085845**

(43) Date of publication of application:
**16.12.2015 Bulletin 2015/51**

(73) Proprietor: **Edan Instruments, Inc.**
**Shenzhen, Guangdong 518067 (CN)**

(72) Inventors:
• **XIANG, Xiaofei**
**Shenzhen**
**Guangdong 518055 (CN)**

• **LU, Yinhui**
**Shenzhen**
**Guangdong 518055 (CN)**
• **ZHAO, Zhixiang**
**Shenzhen**
**Guangdong 518055 (CN)**

(74) Representative: **Prol European Patent Attorneys**
**Postfach 2123**
**90711 Fürth (DE)**

(56) References cited:
EP-A1- 0 545 014     WO-A2-2004/064605
CN-A- 1 127 628      CN-A- 1 244 779
CN-A- 1 906 485      CN-A- 101 889 863
DE-A1-102006 014 825  JP-A- 2005 131 434
US-A- 4 547 735      US-A1- 2006 025 661

**Description**

**FIELD OF THE INVENTION**

The present invention relates to the field of medical instruments, in particular to a detection system for improving accuracy of hematocrit measurement and an operation control method.

**BACKGROUND OF THE INVENTION**

HCT, i.e., hematocrit, is an important parameter in the field of blood analysis. Blood, after anti-coagulation treatment, may be centrifugally separated into two parts: plasma and blood cells. Assumed that blood is centrifuged in a particular test tube (Wintrobe tube) at a specified speed for a specified period of time, eventually making the red blood cells completely packed on the bottom of the test tube. In this case, red blood cells get close to each other to exclude plasma as much as possible, and the plasma is now entirely squeezed onto the blood cells. At this moment, the percentage of red blood cells in the whole blood is the hematocrit, i.e., the volume (or percentage) of the packed red blood cells, also called packed cell volume. Hematocrit is usually abbreviated as HCT. At present, the volume of red blood cells per liter of blood (L/L) is often regarded as the unit of measurement.

HCT is helpful in knowing the increase or reduction of red blood cells. When the absolute value of red blood cells increases due to various reasons, HCT increases accordingly. The reduction of HCT is related to various anemia diseases.

Common abbreviations are HCT, Ht, packed cell volume (PCV)

The reference values are, male: 0.40-0.50 L/L (40%-50%); female: 0.37-0.45 L/L (37%-45%)

In the field of blood analysis, HCT measurement is usually done by AC coupling. An excitation signal is output by an excitation source, and an AC signal value corresponding to a blood impedance is collected by the voltage division of serially-connected standard resistors and blood sample impedance. Due to the serial connection, the signal of the blood is proportional to the impedance. Therefore, the blood impedance is obtained by measuring this signal. In a traditional HCT detection method, the excitation source is a PWM wave. Such a traditional method has many shortcomings although it is simple.

First, the precision of HCT measurement is greatly decreased, because the amplitude of the PWM wave generated by central processing is quite unstable and is likely to drift. The short-term stability of the waveform amplitude is merely about 5%. The change in amplitude will be greater if long-term drift is taken into account.

Second, it is difficult to generate a sine wave of high frequency by central processing. The PWM wave has, when applied to the blood after filtering treatment, an irregular waveform rich in spectrum. Harmonic distortion will be inevitably caused after amplification and conversion, which further increases the HCT detection error. Therefore, such a traditional GCT detection method is merely suitable for occasions without high requirements. If a sine wave is generated by central processing and internal DAC, significant occupation of the central processing resources will be caused due to a too high refresh rate (In order to generate a sine wave 100KHz, according to the DAC method, a smooth sine wave may be formed by drawing at least 50 points within each cycle. That is, the refresh rate of DAC reaches at least 5Msps, one refresh every 0.2µs). Consequently, other programs are unable to work. Thus, it is desirable.

In addition, the traditional HCT detection devices have complex design and poor reliability.

Therefore, in conclusion, it is difficult to achieve precise measurement by the traditional HCT measurement devices. DE102006014825A1 discloses a system for measuring hematocrit, comprising a processing unit connected to a sine wave excitation source that has an output end connected to a blood sample unit. The output of the blood sample unit is communicated to a measurement circuit unit, which amplifies and converts the signal. A signal conversion unit then converts the analog signal to a digital signal and communicates it to the processing unit. Furthermore, CN101889863A discloses a system for measuring hematocrit, the system comprising a single-ended to differential amplifier.

**SUMMARY OF THE INVENTION**

In order to solve the problems of the prior art, the present invention provides a detection system for improving accuracy of hematocrit measurement.

The present invention provides a detection system for improving accuracy of hematocrit measurement, including a central processing unit, an excitation source unit, a blood sample unit, a precise measurement circuit unit and a signal collecting unit; the central processing unit is connected to the excitation source unit and configured to output a control command to the excitation source unit; the excitation source unit is configured to generate a sine wave, and the output end of the excitation source unit is connected to the input end of the blood sample unit; the output end of the blood sample unit is communicated to the input end of the precise measurement circuit unit, and the blood sample unit is configured to collect a blood sample impedance signal; the precise measurement circuit unit is configured to complete amplification of the signal and conversion of the signal to an effective value after the amplification; the signal collecting unit is configured to complete single-ended to differential amplification and analog-digital conversion of a signal of the effective value of the blood impedance; the output end of the precise measurement circuit unit is connected to the signal collecting unit, the central processing unit is connected to the signal collecting unit, and the signal collecting unit outputs the processed data of the effective value of the blood impedance to the central processing unit; and the central processing unit calculates a blood impedance according to the effective value of the blood impedance, and the central processing unit outputs a control command to the signal collecting unit.

In accordance with the present invention, the excitation source unit includes a waveform generator circuit and a waveform converter circuit connected to the waveform generator circuit; the waveform generator circuit is configured to generate a sine wave; and the waveform converter circuit is configured to isolate the DC component of a signal output from the waveform generator circuit and convert a positive sine signal into a positive and negative half-cycle sine wave.

Also in accordance with the present invention, the waveform generator circuit includes a monolithic function generator, a crystal oscillator unit and a precise voltage reference unit, the precise voltage reference unit being connected to a voltage reference interface of the monolithic function generator and configured to provide a precise voltage reference for the monolithic function generator, the monolithic function generator being connected to the crystal oscillator unit, the crystal oscillator unit being configured to provide a high-precision clock signal; and the input end of the waveform converter circuit is connected to the output end of the monolithic function generator, and configured to isolate the DC component of a signal output from the monolithic function generator and convert a positive sine signal into a positive and negative half-cycle sine wave.

In a preferred embodiment, the waveform converter circuit includes a capacitor, a first resistor and a first operational amplifier unit; one end of the capacitor is connected to the output end of the monolithic function generator, and the other end of the capacitor is connected to the resistor and the in-phase input end of the first operational amplifier unit; the other end of the first resistor is grounded; the in-phase end of the first operational amplifier unit is connected to a common node of the capacitor and the first resistor; and one end of a second resistor is connected to the output end of the first operational amplifier unit.

In another preferred embodiment, the blood sample unit is a circuit to be tested; the blood sample unit includes a second resistor, a fifth resistor, an analog switch and a blood equivalent impedance unit; one end of the second resistor is connected to the output end of the excitation source unit; one end of the blood equivalent impedance unit is connected to the second resistor, and the other end of the blood equivalent impedance unit is grounded; one end of the fifth resistor is connected to the second resistor, and the other end of the fifth resistor is connected to one end of the analog switch; the other end of the analog switch is grounded; and both the second resistor and the fifth resistor are standard resistors.

In another preferred embodiment, the precise measurement circuit unit includes a second operational amplifier unit, a third resistor, a fourth resistor and a root mean square converter chip; the in-phase end of the second operational amplifier unit is connected to a common node of the second resistor and the blood equivalent impedance unit, and the out-phase end of the second operational amplifier unit is connected to a common node of the third resistor and the fourth resistor; the output of the second operational amplifier unit is connected to the third resistor and the input end of the root mean square converter chip, and the input end of the root mean square converter chip is connected to a common node of the output end of the second operational amplifier unit and the third resistor; and the other end of the fourth resistor is grounded.

In another preferred embodiment, the signal collecting unit includes a high-resolution analog-digital converter, an analog-digital converter driving circuit and a precise voltage reference unit; the input end of the analog-digital converter driving circuit is connected to the output end of the root mean square converter chip, and the output end of the analog-digital converter driving circuit is connected to the input end of the high-resolution analog-digital converter; the high-resolution analog-digital converter is connected to the output end of the precise voltage reference unit; and the high-resolution analog-digital converter is connected to the central processing unit.

In another preferred embodiment, the monolithic function generator is a direct digital frequency synthesizer which is produced by the AD Company (model: AD9832); and the high-resolution analog-digital converter is a Delta-Sigma analog-digital converter.

In another preferred embodiment, there are two precise voltage reference units, respectively a first precise voltage reference unit and a second precise voltage reference unit; the first precise voltage reference unit is connected to the voltage reference interface of the monolithic function generator and configured to provide a precise voltage reference for the monolithic function generator; and the high-resolution analog-digital converter is connected to the output end of the second precise voltage reference unit.

The present invention further provides an operation control method for a detection system, including the steps defined in claim 8.

In a preferred embodiment, the method further comprises the following steps:

B. setting a standard value: turning an analog switch off, not accessing a blood equivalent impedance unit to the blood, and placing a standard resistor for scaling into the blood equivalent impedance unit;

C. scaling a circuit: setting M scaling points within a typical impedance measurement range from 1K to 15K, where M is greater than or equal to 2, starting the circuit once for scaling each time when one standard resistor is placed to the blood equivalent impedance unit in step B, and repeatedly executing step B and step C for M times; at the end of scaling, saving a code value obtained by ADC (analog-digital conversion) corresponding to each of the standard resistors into an internal memory of the central processing unit to obtain a correspondence between the code value obtained by ADC and the standard resistor;

D. back-testing of the scaling: accessing a resistor having a resistance of R to the original blood equivalent impedance unit Rx, judging whether the measurement deviation exceeds a preset deviation value, if so, returning to step B; and if not, executing step E, where R is from 1KΩ to 10KΩ; and

E: circuit self-detection: turning the analog switch on, accessing the fifth resistor to the measurement circuit to measure the resistance of the fifth resistor, judging whether the error of the fifth resistor exceeds a preset value, if so, giving a prompt indicative of circuit abnormality and automatically terminating the measurement, and if not, performing the detection task by the detection system.

The present invention has the beneficial effects that the HCT measurement precision is improved by generating a sine wave by the excitation source unit and performing control by the central processing unit; the detection system of the present invention is simple and reliable, and implements precise measurement.

## BRIEF DESCRIPTION OF THE DRAWINGS

Fig. 1 is a principle block diagram of a detection system;
Fig. 2 is a principle diagram of an embodiment of the detection system according to the present invention;
Fig. 3 is a flowchart of an operation control method; and
Fig. 4 is a diagram of circuit scaling.

## DETAILED DESCRIPTION OF THE PREFERRED EMBODIMENTS

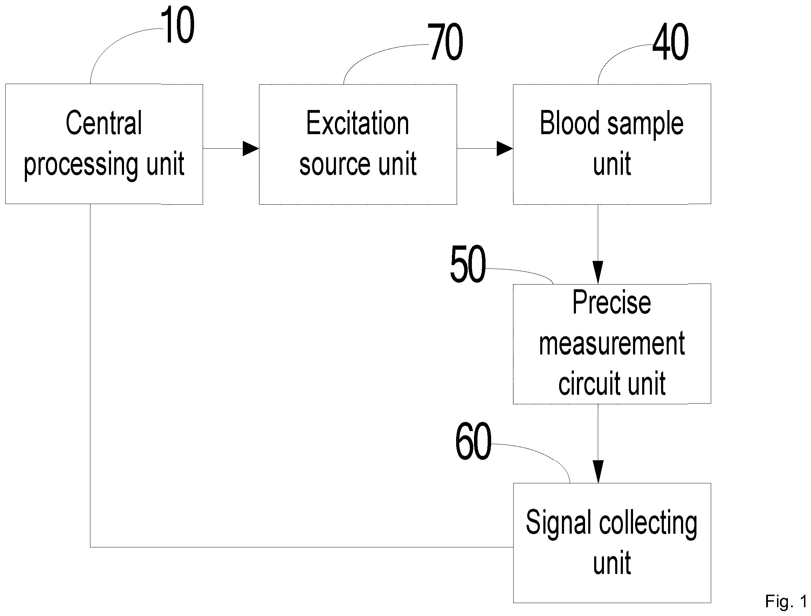

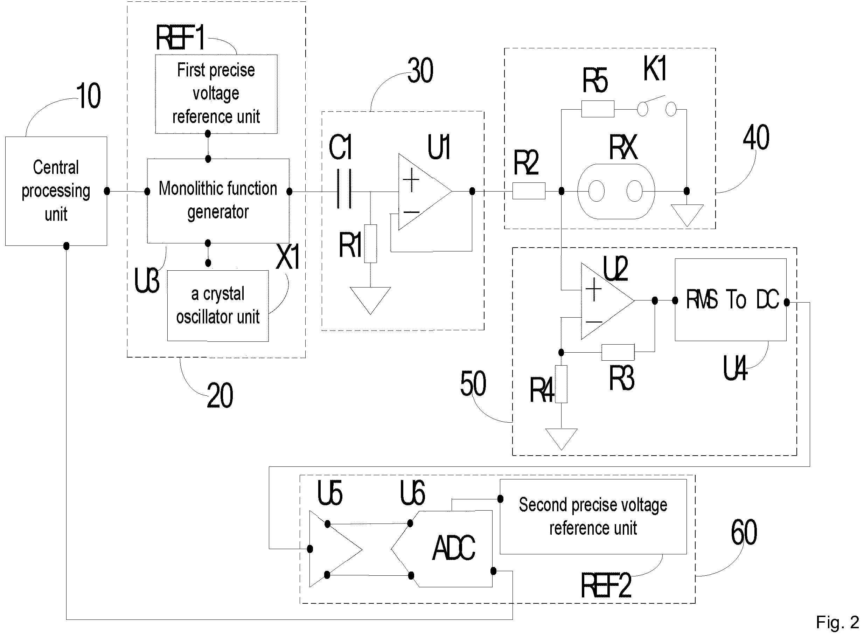

As shown in Fig. 1 and Fig. 2, the present invention provides a detection system for improving accuracy of hematocrit measurement, including a central processing unit 10, an excitation source unit 70, a blood sample unit 40, a precise measurement circuit unit 50 and a signal collecting unit 60; the central processing unit 10 is connected to the excitation source unit 70 and configured to output a control command to the excitation source unit 70; the excitation source unit 70 is configured to generate a sine wave, and the output end of the excitation source unit 70 is connected to the input end of the blood sample unit 40; the output end of the blood sample unit 40 is communicated to the input end of the precise measurement circuit unit 50, and the blood sample unit 40 is configured to collect a blood sample impedance signal; the precise measurement circuit unit 50 is configured to complete amplification of the signal and conversion of the signal to an effective value after the amplification; the signal collecting unit 60 is configured to complete single-ended to differential amplification and analog-digital conversion of a signal of the effective value of the blood impedance; the output end of the precise measurement circuit unit 50 is connected to the signal collecting unit 60, the central processing unit 10 is connected to the signal collecting unit 60, and the signal collecting unit 60 outputs the processed data of the effective value of the blood impedance to the central processing unit 10; and the central processing unit 10 calculates a blood impedance according to the effective value of the blood impedance, and the central processing unit 10 outputs a control command to the signal collecting unit 60.

The central processing unit 10 is connected to the monolithic function generator U3 through an SPI interface. Of course, the central processing unit 10 may be communicated to the monolithic function generator U3 through a 12C or GPIO.

The excitation source unit 70 includes a waveform generator circuit 20 and a waveform converter circuit 30; the waveform generator circuit 20 includes a monolithic function generator U3, a crystal oscillator unit X1 and a precise voltage reference unit; the first precise voltage reference unit REF1 is connected to the voltage reference interface of the monolithic function generator U3 and configured to provide a precise voltage reference for the monolithic function generator U3; the monolithic function generator U3 is connected to the crystal oscillator unit X1 and the crystal oscillator unit X1 is configured to provide a high-precision clock signal; and the input end of the waveform converter circuit 30 is connected to the output end of the monolithic function generator U3, and the waveform converter circuit 30 is configured to isolate the DC component of a signal output from the monolithic function generator U3 and convert a positive sine signal into a positive and negative half-cycle sine wave.

As the monolithic function generator U3 is connected to the crystal oscillator unit X1 and the crystal oscillator unit X1 is configured to provide a high-precision clock signal, the frequency of the output waveform may be made quite stable. The monolithic function generator U3 is a programmable monolithic function generator which has a waveform update rate up to 25MHz and can generate a quite ideal sine wave. The monolithic function generator U3 is a direct digital frequency synthesizer which is produced by the AD Company model: AD9832, its output waveform amplitude is determined by commands input by the first precise voltage reference unit REF1 and the central processing unit 10 and its output waveform frequency is determined by the crystal oscillator unit X1.

The waveform converter circuit 30 includes a capacitor C1, a first resistor R1 and a first operational amplifier unit U1; one end of the capacitor C1 is connected to the output end of the monolithic function generator U3, and the other end of the capacitor C1 is connected to the resistor R1 and the in-phase input end of the first operational amplifier unit U1; the other end of the first resistor R1 is grounded; and the in-phase end of the first operational amplifier unit U1 is connected to a common node of the capacitor C1 and the first resistor R1.

The blood sample unit 40 is a circuit to be tested; the blood sample unit 40 includes a second resistor R2, a fifth resistor R5, an analog switch K1 and a blood equivalent impedance unit RX; one end of the second resistor R2 is connected to the output end of first operational amplifier unit U1; one end of the second resistor R2 is connected to the output end of the excitation source unit 70; one end of the blood equivalent impedance unit RX is connected to the second resistor R2, and the other end of the blood equivalent impedance unit RX is grounded; one end of the fifth resistor R5 is connected to the second resistor R2, and the other end of the fifth resistor R5 is connected to one end of the analog switch K1; the other end of the analog switch K1 is grounded; and both the second resistor R2 and the fifth resistor R5 are standard resistors.

The second resistor R2 is used for realizing voltage division with the blood impedance, to attenuate the AC signal strength on the blood, in order to avoid unnecessary chemical reactions. The fifth resistor R5 and the analog switch K1 are used for self-detection of the channels to be tested. This circuit is self-detected every time of blood measurement and this ensures the measurement reliability of this device. Due to the serial connection between the blood impedance, the signal amplitude on the blood and the corresponding impedance form a quadratic function. In the present invention, by multi-point scaling and performing piecewise linear treatment, a broken line approaching to an ideal curve is obtained. The precision of calculation is greatly improved while simplifying the calculation.

The precise measurement circuit unit 50 includes a second operational amplifier unit U2, a third resistor R3, a fourth resistor R4 and a root mean square converter chip U4; the in-phase end of the second operational amplifier unit U2 is connected to a common node of the second resistor R2 and the blood equivalent impedance unit RX, and the out-phase end of the second operational amplifier unit U2 is connected to a common node of the third resistor R3 and the fourth resistor R4; the output of the second operational amplifier unit U2 is connected to the third resistor R3 and the input end of the root mean square converter chip U4, and the input end of the root mean square converter chip U4 is connected to a common node of the output end of the second operational amplifier unit U2 and the third resistor R3; and the other end of the fourth resistor R4 is grounded.

In the root mean square converter chip U4, the RMS (root mean square) chip is quite stable, thereby overcoming the temperature drift problem of a conventional diode rectifier circuit, the non-linear problem and the long-term stability problem.

The function of the root mean square converter chip U4 is to complete the root mean square conversion of the input signal and output a DC signal having a size exactly equal to the effective value of the input AC signal. The root mean square converter chip U4 employed in this embodiment has a linearity up to 0.02%, which can ensure a quite high detection precision. This index is extremely important to the measurement of the effective value, and can not be calibrated or eliminated.

The signal collecting unit 60 includes a high-resolution analog-digital converter U6, an analog-digital converter driving circuit U5 and a second precise voltage reference unit REF2; the input end of the analog-digital converter driving circuit U5 is connected to the output end of the root mean square converter chip U4 , and the output end of the analog-digital converter driving circuit U5 is connected to the input end of the high-resolution analog-digital converter U6; the high-resolution analog-digital converter U6 is connected to the output end of the second precise voltage reference unit REF2; and the high-resolution analog-digital converter U6 is connected to the central processing unit 10.

The first precise voltage reference unit REF1 and the second precise voltage reference unit REF2 may be combined. This may also achieve the purpose of connecting to the voltage reference interface of the monolithic function generator U3 and to the high-resolution analog-digital converter U6.

The analog-digital converter driving circuit U5 completes single-ended to differential amplification and the signal gain is doubled. With regard to the high-resolution analog-digital converter U6, as the collected signal is a DC signal, the conversion precision is much required than the conversion rate. Therefore, as a preferred implementation, the high-resolution analog-digital converter U6 is a Delta-Sigma analog-digital converter. The precision of signal collection and conversion of the high-resolution analog-digital converter U6 is determined by the ADC valid bits of the high-resolution analog-digital converter U6 and the precision of the second precise voltage reference unit REF2, which may both reach a high precision. In a preferred embodiment, the high-resolution analog-digital converter U6 is 24-bit, and the typical value of the valid bits of the high-resolution analog-digital converter U6 may reach 21.5dB. The initial voltage precision of the voltage reference is 0.06%. Due to the gain error of the high-resolution analog-digital converter U6, and as both the imbalance and the initial precision of the voltage reference may be eliminated during calibration, the error of the signal collecting unit is mainly resulted from the temperature drift of the high-resolution analog-digital converter U6. The temperature drift reference depends upon the long-term stability. In this embodiment, the temperature drift of the high-resolution analog-digital converter U6 is 2ppm/°C, an extremely low value which can be completely ignored, when compared with the temperature drift reference of 9ppm/°C. When the temperature changes by 16°C, change in the temperature drift reference is just 144ppm, and the long-term drift reference is below 50ppm. Therefore, the error of the measurement circuit may be controlled at 200ppm, i.e., within 0.02%.

The central processing unit 10 is connected to the high-resolution analog-digital converter U6 through an SPI interface. Of course, the central processing unit 10 may be communicated to the high-resolution analog-digital converter U6 through a 12C or GPIO.

The excitation source unit 70 may be an active crystal-oscillation frequency-division or high-speed digital-analog converter. The RMS chip may be replaced by a high-resolution analog-digital converter.

A standard sine wave is generated by the first precise voltage reference unit REF1 in cooperation with the monolithic function generator U3 (DDS), direct digital frequency synthesizer, the amplitude is quite stable and completely controlled by the first precise voltage reference unit REF1, and the precision of the amplitude may be within 0.02%, so that the HCT measurement error caused by the amplitude drift of the excitation source may be significantly solved; meanwhile, with the precise root mean square converter chip U4, the conversion may realize a precision within 0.15%, with simple signal chains and fewer links; two key links which may influence the measurement precision, i.e., the error of the excitation source and the error of the measurement circuit, are easily controlled, so that the measurement precision of the present invention is far greater than that of a conventional measurement technology and can be within 0.2%. Furthermore, due to a self-detection function, the measurement is quite reliable without the risk of resulting incorrect measurement data from a circuit failure.

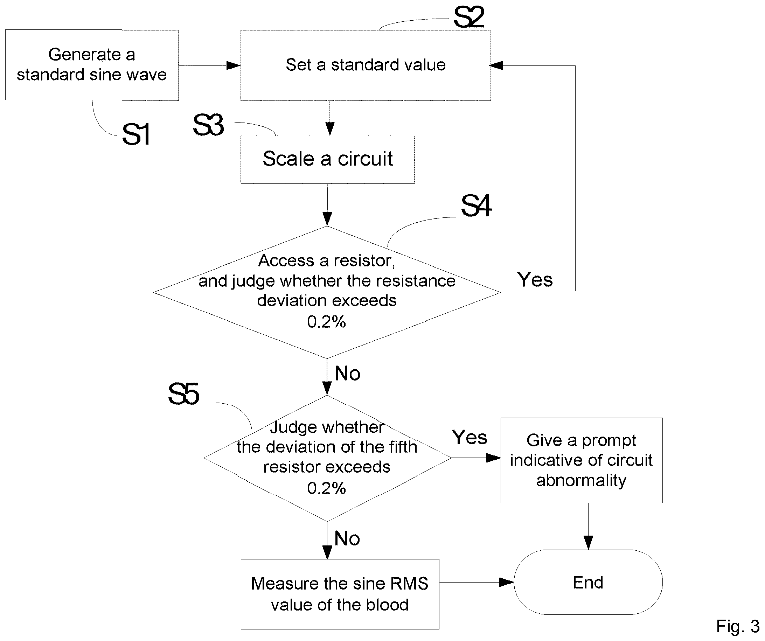

As shown in Fig. 3, an operation control method for the detection system of the present invention may include the following S1 to S5:

S1: generating a standard sine wave: outputting, by a central processing unit, an instruction to a monolithic function generator to control the monolithic function generator to generate a standard sine wave;

S2: setting a standard value: turning an analog switch off, not accessing a blood equivalent impedance unit to the blood, and placing a standard resistor for scaling into the blood equivalent impedance unit;

S3: scaling a circuit: setting M scaling points within a typical impedance measurement range from 1K to 15K, where M is greater than or equal to 2, starting the circuit once for scaling each time when one standard resistor is placed to the blood equivalent impedance unit in step B, and repeatedly executing step B and step C for M times; at the end of scaling, saving a code value obtained by ADC (analog-digital conversion) corresponding to each of the standard resistors into an internal memory of the central processing unit to obtain a correspondence between the code value obtained by ADC and the standard resistor;

S4: back-testing of the scaling: accessing a 1-10K resistor to the original blood equivalent impedance unit Rx the blood is not accessed to the circuit, measuring whether it is 1-10K, judging whether the measurement deviation exceeds a preset deviation value, if so, determining that the scaling does not pass and returning to S2; and if not, proceeding to S5;

where, in S4, the 1-10K resistor is preferably a 5K resistor and the preset deviation value is preferably 0.2%;

S5: circuit self-detection: turning the analog switch on, accessing the fifth resistor to the measurement circuit to measure the resistance of the fifth resistor, judging whether the error of the fifth resistor exceeds a preset value, if so, giving a prompt indicative of circuit abnormality and automatically terminating the measurement, and if not, performing the detection task by the detection system;

where, in S5, the preset value is preferably 0.2%.

An embodiment of the operation control method will be described below.

In S1, the central processing unit is a single-chip computer, the monolithic function generator is a direct digital frequency synthesizer, and the single-chip computer outputs an instruction to the direct digital frequency synthesizer to control the direct digital frequency synthesizer to generate a standard sine wave.

In S2, the analog switch is turned off, the blood is not accessed, and a standard resistor for scaling is placed to the blood equivalent impedance unit.

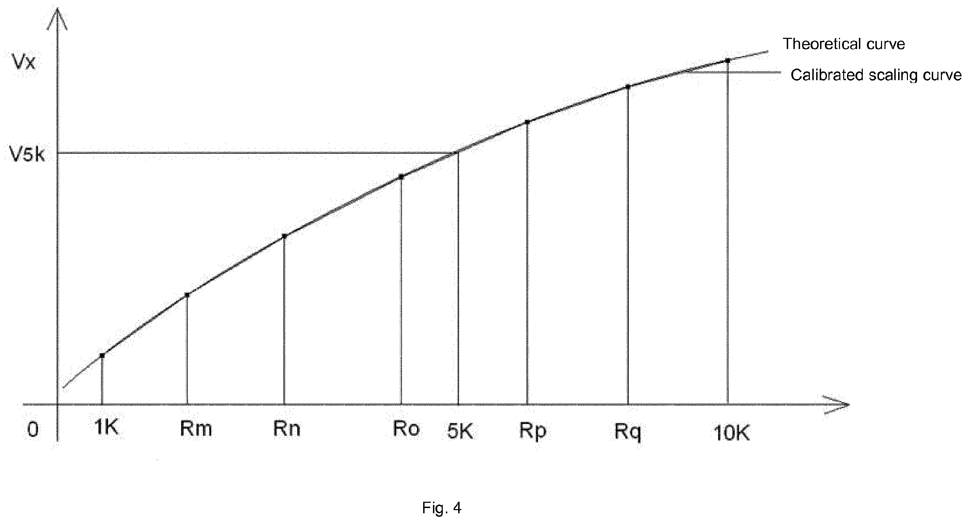

In S3, five scaling points are set within a typical impedance measurement range from 1K to 15K, S2 and S3 are repeatedly executed for five times, one scaling is started each time when one standard resistor is placed to the blood equivalent impedance unit in S2; at the end of scaling, a code value obtained by ADC (LSBm, LSBn, LSBo, LSBp, LSBq) corresponding to each of the standard resistors (Rm, Rn, Ro, Rp, Rq) into an internal memory of the single-chip computer to obtain a correspondence between the code value obtained by ADC (LSBm, LSBn, LSBo, LSBp, LSBq) and the standard resistor (Rm, Rn, Ro, Rp, Rq). As shown in Fig. 4, five straight lines may be obtained according to the correspondences described above, and the intersection points of adjacent straight lines are the five scaling points.

In S4, the way of modifying the scaling parameter may be increasing or decreasing the sine amplitude output by the DDS.

In S5, the analog switch is turned on, and the fifth resistor is thus accessed to the measurement circuit. Now, due to the self-detection, the blood is not accessed to the loop, there is thus no blood equivalent impedance unit and the resistance of the fifth resistor is not one of the resistances at the five scaling points. Therefore, whether the circuit works normally and whether the measurement precision of the circuit reaches the requirement both may be detected. If the error of the fifth resistor does not exceed 0.2%, the RMS value of the sine signal on the blood is measured. The RMS value of the sine signal on the blood is obtained through the codes obtained by ADC. By the scaling curve, the blood impedance value is calculated and the blood HCT value is then calculated.

The principle of measurement and scaling will be discussed below.

Assumed that the blood impedance to be tested is Rx, the amplitude of the excitation source is Vp, the partial voltage on the blood equivalent impedance is Vx, the input signal of RMS is Vsine, the output signal is Vdc, the code value obtained by ADC is LSB, and the ADC voltage reference VREF=5V, then:

$$\frac{Vx}{Vp} \quad \frac{Rx}{Rx+R2} \text{-----------------------------------------} (1)$$

$$Vdc = \frac{\sqrt{2}}{2}V\sin e = \frac{\sqrt{2}}{2}*10*Vx \text{----------------------} (2)$$

$$2*Vdc = \frac{5V}{2^{24}-1}*LSB \text{--------------------------------} (3)$$

Vdc may be obtained from Formula (3) and then substituted into Formula (2) to obtain Vx, and Vx is then substituted into Formula (1). As Vp is a known, Rx may be obtained from Formula (1).

However, as Formula (1) is a quadratic function, the operation is complex. Therefore, in the present invention, multi-point scaling is used. For example, five points (Rm, Rn, Ro, Rp, Rq) are inserted from 1K to 15K to divide this range into six sections, with the value of each interval being far less than the serially-connected resistor R2, as shown in Fig. 4. Accordingly, each section may be regarded to be linear. Five straight lines may be produced by the multi-point scaling. The straight line, on which a code value collected by ADC is, may be determined. In this way, the accurate Rx value may be obtained quickly. As shown in Fig. 4, after the multi-point scaling, the corrected scaling curve is substantially coincided with the theoretical curve, thereby realizing high precision.

By multi-point scaling and performing piecewise linear treatment, a single correspondence is established between the resistor Rx to be tested and the code value obtained by ADC. In this way, the constant errors in the intermediate links, for example, the initial precision of the references REF1 and REF2, the imbalance between the ADC and the operational amplification, and the gain error of the ADC, may be eliminated. The dependence of the system on those parameters is reduced greatly. A high precision may be maintained for a circuit as long as there is no drift.

The CPU inputs a command, according to the program settings, to the monolithic function generator U3. Under the precise voltage reference, U3 outputs a sine wave with an amplitude of 1Vpp. This sine wave has a crest of 1V and a trough of 0V. This signal is passed to C1 to be converted into a sine wave of ±500mVpp, then buffered by U1, and finally directly applied to the serially-connected resistor R2 and the blood equivalent impedance RX. In a preferred embodiment, R2 has a resistance of 98Kohm, and the blood equivalent impedance is usually from 2Kohm to 10Kohm.

Therefore,
at a minimum blood capacitive impedance,
the sine wave component on RX : ±500mVpp*2K(98K+2K) = ±10mVpp;
at a maximum blood capacitive impedance,
the sine wave component on RX: ±500mVpp*10K(98K+10K) = ±46.3mVpp
Signal of RX is passed to U2 to be amplified by twenty times, i.e., ±200mVpp to ±926mVpp.

Therefore, when the blood impedance changes within a maximum range from 2Kohm to 10Kohm, the input signal of the RMS chip is 02V to 0.926V, exactly within a range having a very good linearity, so that this input signal may be highly precisely converted into a DC signal by the RMS chip. The precision level may reach 0.15%. As the signal output by the RMS has been up to hundreds of millivolts, the precision of ADC collection may reach 0.02%. Accordingly, the whole measurement circuit may realize a precision of 0.2%, which is far greater than that of the traditional solutions. Meanwhile, the use of the self-detection circuit greatly improves the reliability of the measurement circuit device.

By using a stable DDS as the signal source and RMS as the detection chip, reliable working and excellent performance are realized, and the precision of blood impedance detection is substantively improved (may reach 0.2%). The deficiencies, i.e., low precision, large error and complex circuit, of the traditional HCT detection circuits are thoroughly solved.

The foregoing is just further detailed description of the present invention with specific preferred implementations. It should not be considered that the specific implementation of the present invention is limited thereto. For a person of ordinary skill in the art, various simple deductions and replacements may be made without departing from the protection scope of the present invention, as defined by the appended claims.

## Claims

1. A detection system for improving accuracy of hematocrit measurement, comprising a central processing unit (10), an excitation source unit (70), a blood sample unit (40), a precise measurement circuit unit (50) and a signal collecting unit (60);

   wherein the central processing unit (10) is connected to an input end of the excitation source unit (70), an output end of the excitation source unit (70) is connected to an input end of the blood sample unit (40), wherein an output end of the blood sample unit (40) is connected to an input end of the precise measurement circuit unit (50), wherein an output end of the precise measurement circuit unit (50) is connected to the signal collecting unit (60), and wherein the central processing unit (10) is connected to the signal collecting unit (60);

   wherein the central processing unit (10) is configured to output an instruction to the excitation source unit (70); and the excitation source unit (70) is configured to generate a standard sine wave according to the instruction;

   wherein the blood sample unit (40) is configured to collect a blood sample impedance signal indicating a blood impedance;

   wherein the precise measurement circuit unit (50) is configured to receive the blood sample impedance signal from the blood sample unit (40) and complete amplification (U2) of the blood sample impedance signal and conversion (U4) of the blood sample impedance signal to an effective value after the amplification, to thus provide a signal indicating the effective value of the blood impedance;

   wherein the signal collecting unit (60) is configured to receive the signal indicating the effective value of the blood impedance from the precise measurement circuit unit (50) and complete single-ended to differential amplification (U5) and analog-digital conversion (U6) of the signal indicating the effective value of the blood impedance, to thus provide a processed data indicating the effective value of the blood impedance, and the signal collecting unit (60) is configured to output the processed data indicating the effective value of the blood impedance to the central processing unit (10); and

   wherein the central processing unit (10) is further configured to calculate a blood impedance value according to the processed data indicating the effective value of the blood impedance and to output a control command to the signal collecting unit (60);

   wherein the excitation source unit (70) comprises a waveform generator circuit (20) and a waveform converter circuit (30) connected to the waveform generator circuit (20) wherein the waveform generator circuit (20) is configured to generate the standard sine wave and the waveform converter circuit (30) is configured to isolate a DC component of a signal output from the waveform generator circuit (20) and convert a positive sine signal into a positive and a negative half-cycle sine waves;

   characterized in that the waveform generator circuit (20) comprises a monolithic function generator (U3) configured to generate the standard sine wave;

   wherein the waveform generator circuit (20) further comprises a crystal oscillator unit (X1) and a precise voltage reference unit, the precise voltage reference unit being connected to a voltage reference interface of the monolithic function generator (U3) and configured to provide a precise voltage reference for the monolithic function generator (U3), the monolithic function generator (U3) being connected to the crystal oscillator unit (X1), the crystal oscillator unit (X1) being configured to provide a high-precision clock signal; and the input end of the waveform converter circuit (30) is connected to the output end of the monolithic function generator (U3), and configured to isolate the DC component of a signal output from the monolithic function generator (U3) and convert the positive sine signal into the positive and negative half-cycle sine wave.

**2.** The detection system according to claim 1, wherein the waveform converter circuit (30) comprises a capacitor (C1), a first resistor (R1) and a first operational amplifier unit (U1); one end of the capacitor (C1) is connected to the output end of the monolithic function generator (U3), and the other end of the capacitor (C1) is connected to the resistor (R1) and the in-phase input end of the first operational amplifier unit (U1); the other end of the first resistor (R1) is grounded; the in-phase end of the first operational amplifier unit (U1) is connected to a common node of the capacitor (C1) and the first resistor (R1); and one end of a second resistor (R2) is connected to the output end of the first operational amplifier unit (U1).

**3.** The detection system according to claim 2, wherein the blood sample unit (40) is a circuit to be tested; the blood sample unit (40) comprises a second resistor (R2), a fifth resistor (R5), an analog switch (K1) and a blood equivalent impedance unit (RX); one end of the second resistor (R2) is connected to the output end of the excitation source unit (70); one end of the blood equivalent impedance unit (RX) is connected to the second resistor (R2), and the other end of the blood equivalent impedance unit (RX) is grounded; one end of the fifth resistor (R5) is connected to the second resistor (R2), and the other end of the fifth resistor (R5) is connected to one end of the analog switch (K1); the other end of the analog switch (K1) is grounded; and both the second resistor (R2) and the fifth resistor (R5) are standard resistors.

**4.** The detection system according to claim 3, wherein the precise measurement circuit unit (50) comprises a second operational amplifier unit (U2), a third resistor (R3), a fourth resistor (R4) and a root mean square converter chip (U4); the in-phase end of the second operational amplifier unit (U2) is connected to a common node of the second resistor (R2) and the blood equivalent impedance unit (RX), and the out-phase end of the second operational amplifier unit (U2) is connected to a common node of the third resistor (R3) and the fourth resistor (R4); the output of the second operational amplifier unit (U2) is connected to the third resistor (R3) and the input end of the root mean square converter chip (U4), and the input end of the root mean square converter chip (U4) is connected to a common node of the output end of the second operational amplifier unit (U2) and the third resistor (R3); and the other end of the fourth resistor (R4) is grounded.

**5.** The detection system according to claim 4, wherein the signal collecting unit (60) comprises a high-resolution analog-digital converter (U6), an analog-digital converter driving circuit (U5) and a precise voltage reference unit; the input end of the analog-digital converter driving circuit (U5) is connected to the output end of the root mean square converter chip (U4), and the output end of the analog-digital converter driving circuit (U5) is connected to the input end of the high-resolution analog-digital converter (U6); the high-resolution analog-digital converter (U6) is connected to the output end of the precise voltage reference unit; and the high-resolution analog-digital converter (U6) is connected to the central processing unit (10).

**6.** The detection system according to claim 5, wherein the monolithic function generator (U3) is a direct digital frequency synthesizer which is produced by the AD Company (model: AD9832); and the high-resolution analog-digital converter (U6) is a Delta-Sigma analog-digital converter.

**7.** The detection system according to claim 6, wherein there are two precise voltage reference units, respectively a first precise voltage reference unit (REF1) and a second precise voltage reference unit (REF2); the first precise voltage reference unit (REF1) is connected to the voltage reference interface of the monolithic function generator (U3) and configured to provide a precise voltage reference for the monolithic function generator (U3); and the high-resolution analog-digital converter (U6) is connected to the output end of the second precise voltage reference unit (REF2).

**8.** A method of operating the detection system according to claim 1, comprising the following steps:

generating a standard sine wave, which comprises: outputting, by the central processing unit (10), an instruction to the excitation source unit (70) to generate the standard sine wave;
collecting, by the blood sample unit (40), a blood sample impedance signal indicating a blood impedance;
completing, by the precise measurement circuit unit (50), amplification (U2) of the blood sample impedance signal and conversion (U4) of the blood sample impedance signal to an effective value after the amplification, to thus provide a signal indicating the effective value of the blood impedance;
completing, by the signal collecting unit (60), single-ended to differential amplification (U5) and analog-digital conversion (U6) of the signal indicating the effective value of the blood impedance, to thus provide a processed data indicating the effective value of the blood impedance;
calculating, by the central processing unit (10), a blood impedance value according to the processed data indicating the effective value of the blood impedance;

**characterized in that** the standard sine wave is generated by the monolithic function generator (U3) comprised in the waveform generator circuit (20) comprised in the excitation source unit (70);

wherein the waveform converter circuit (30) further isolates a DC component of a signal output from the waveform generator circuit (20) and converts a positive sine signal into a positive and a negative half-cycle sine waves;

wherein the precise voltage reference unit further provides a precise voltage reference for the monolithic function generator (U3), the crystal oscillator unit (X1) provides a high-precision clock signal, and the waveform converter circuit (30) isolates the DC component of a signal output from the monolithic function generator (U3) and converts the positive sine signal into the positive and negative half-cycle sine waves.

9. The method according to claim 8, further comprising the following steps:

B) setting a standard value: turning an analog switch off, not accessing a blood equivalent impedance unit to the blood, and placing a standard resistor for scaling into the blood equivalent impedance unit;

C) scaling a circuit: setting M scaling points within a typical impedance measurement range from 1K to 15K, where M is greater than or equal to 2, starting the circuit once for scaling each time when one standard resistor is placed to the blood equivalent impedance unit in step B, and repeatedly executing step B and step C for M times; at the end of scaling, saving a code value obtained by ADC (analog-digital conversion) corresponding to each of the standard resistors into an internal memory of the central processing unit to obtain a correspondence between the code value obtained by ADC and the standard resistor;

D) back-testing of the scaling: accessing a resistor having a resistance of R to the original blood equivalent impedance unit Rx, judging whether the measurement deviation exceeds a preset deviation value, if so, returning to step B; and if not, executing step E, where R is from $1K\Omega$ to $10K\Omega$; and

E) circuit self-detection: turning the analog switch on, accessing the fifth resistor to the measurement circuit to measure the resistance of the fifth resistor, judging whether the error of the fifth resistor exceeds a preset value, if so, giving a prompt indicative of circuit abnormality and automatically terminating the measurement, and if not, performing the detection task by the detection system.

## Patentansprüche

1. Detektionssystem zur Verbesserung der Genauigkeit der Hämatokritmessung, mit einer zentralen Verarbeitungseinheit (10), einer Anregungsquelleneinheit (70), einer Blutprobeneinheit (40), einer präzisen Messkreiseinheit (50) und einer Signalsammeleinheit (60);

wobei die zentrale Verarbeitungseinheit (10) mit einem Eingangsende der Anregungsquelleneinheit (70) verbunden ist, wobei ein Ausgangsende der Erregungsquelleneinheit (70) mit dem Eingangsende der Blutprobeneinheit (40) verbunden ist, wobei ein Ausgabeende der Blutprobeneinheit (40) mit dem Eingabeende der genauen Messschaltkreiseinheit (50) verbunden ist, wobei das Ausgangsende der genauen Messschaltkreiseinheit (50) mit der Signalsammeleinheit (60) verbunden ist, und wobei die zentrale Verarbeitungseinheit (10) mit der Signalsammeleinheit (60) verbunden ist;

wobei die zentrale Verarbeitungseinheit (10) ausgebildet ist, einen Steuerbefehl an die Erregungsquelleneinheit (70) auszugeben; und die Erregungsquelleneinheit (70) ist konfiguriert, entsprechend dem Steuerbefehl eine Sinuswelle zu erzeugen;

wobei die Blutprobeneinheit (40) konfiguriert ist, ein Blutprobenimpedanzsignal zu sammeln, welches die Blutimpedanz anzeigt;

wobei die präzise Messschaltungseinheit (50) ist konfiguriert, das Blutprobenimpedanzsignal von der Blutprobeneinheit (40) zu empfangen und die Verstärkung (U2) des Blutprobenimpedanzsignals und die Umwandlung des Blutprobenimpedanzsignals auf einen effektiven Wert nach der Verstärkung zu vervollständigen, um so ein Signal zur Verfügung zu stellen, welches den effektiven Wert der Blutimpedanz anzeigt;

wobei die Signalsammeleinheit (60) konfiguriert ist, von der präzisen Messschaltungseinheit (50) das Signal zu empfangen, welches den effektiven Wert der Blutimpedanz anzeigt, und eine Eintakt-zu-Differenzverstärkung (U5) und eine Analog-Digital-Umwandlung (U6) des Signals zu vervollständigen, welches den effektiven Wert der Blutimpedanz anzeigt, um somit verarbeitete Daten zur Verfügung zu stellen, welche den effektiven Wert der Blutimpedanz anzeigen, wobei die Signalsammeleinheit (60) die verarbeiteten Daten des Effektivwerts der Blutimpedanz an die zentrale Verarbeitungseinheit (10) ausgibt; und

wobei die zentrale Verarbeitungseinheit (10) ferner dazu ausgebildet ist, einen Blutimpedanzwert gemäß den verarbeiteten Daten zu berechnen, die den effektiven Wert der Blutimpedanz anzeigen, und einen Steuerbefehl an die Signalsammeleinheit (60) auszugeben;

wobei die Anregungsquelleneinheit (70) eine Wellenformgeneratorschaltung (20) und eine Wellenformwandlerschaltung (30) umfasst, die mit der Wellenformgeneratorschaltung (20) verbunden ist; die Wellenformgeneratorschaltung (20) ist konfiguriert, um eine Sinuswelle zu erzeugen; und die Wellenformwandlerschaltung (30) so konfiguriert ist, dass sie die Gleichstromkomponente eines von der Wellenformgeneratorschaltung (20) ausgegebenen Signals isoliert und ein positives Sinussignal in eine positive und negative Halbzyklus-Sinuswelle umwandelt,
**dadurch gekennzeichnet, dass**
die Wellenformgeneratorschaltung (20) einen monolithischen Funktionsgenerator (U3), welcher dazu ausgebildet ist, eine Sinuswelle zu erzeugen,
wobei die Wellenformgeneratorschaltung (20) ferner eine Kristalloszillatoreinheit (X1) und eine genaue Spannungsreferenzeinheit aufweist, wobei die genaue Spannungsreferenzeinheit mit einer Spannungsreferenz verbunden ist Schnittstelle des monolithischen Funktionsgenerators (U3) und konfiguriert, um eine präzise Spannungsreferenz für den monolithischen Funktionsgenerator (U3) bereitzustellen, wobei der monolithische Funktionsgenerator (U3) mit der Kristalloszillatoreinheit (X1) verbunden ist, wobei der Kristalloszillatoreinheit (X1) konfiguriert ist, um ein hochpräzises Taktsignal bereitzustellen; und das Eingangsende der Wellenformwandlerschaltung (30) ist mit dem Ausgangsende des monolithischen Funktionsgenerators (U3) verbunden und konfiguriert, um die Gleichstromkomponente eines von dem monolithischen Funktionsgenerator (U3) ausgegebenen Signals zu isolieren und ein positives Sinussignal in eine positive und negative Halbzyklus-Sinuswelle zu wandeln.

2. Detektionssystem nach Anspruch 1, wobei die Wellenformwandlerschaltung (30) einen Kondensator (C1), einen ersten Widerstand (R1) und eine erste Operationsverstärkereinheit (U1) umfasst; ein Ende des Kondensators (C1) ist mit dem Ausgangsende des monolithischen Funktionsgenerators (U3) verbunden, und das andere Ende des Kondensators (C1) ist mit dem Widerstand (R1) und dem phasengleichen Eingangsende der ersten Operationsverstärkereinheit (U1) verbunden; das andere Ende des ersten Widerstands (R1) ist geerdet; das phasengleiche Ende der ersten Operationsverstärkereinheit (U1) ist mit einem gemeinsamen Knoten des Kondensators (C1) und des ersten Widerstands (R1) verbunden; und ein Ende eines zweiten Widerstands (R2) ist mit dem Ausgangsende der ersten Operationsverstärkereinheit (U1) verbunden.

3. Detektionssystem nach Anspruch 2, wobei die Blutprobeneinheit (40) eine zu testende Schaltung ist; die Blutprobeneinheit (40) umfasst einen zweiten Widerstand (R2), einen fünften Widerstand (R5), einen Analogschalter (K1) und eine Blutäquivalenzimpedanzeinheit (RX); ein Ende des zweiten Widerstands (R2) ist mit dem Ausgangsende der Erregungsquelleneinheit (70) verbunden; ein Ende der Blutäquivalenzimpedanzeinheit (RX) ist mit dem zweiten Widerstand (R2) verbunden, und das andere Ende der Blutäquivalenzimpedanzeinheit (RX) ist geerdet; ein Ende des fünften Widerstands (R5) ist mit dem zweiten Widerstand (R2) verbunden, und das andere Ende des fünften Widerstands (R5) ist mit einem Ende des Analogschalters (K1) verbunden; das andere Ende des Analogschalters (K1) ist geerdet; und sowohl der zweite Widerstand (R2) als auch der fünfte Widerstand (R5) sind Standardwiderstände.

4. Detektionssystem nach Anspruch 3, wobei die präzise Messschaltungseinheit (50) eine zweite Operationsverstärkereinheit (U2), einen dritten Widerstand (R3), einen vierten Widerstand (R4) und einen quadratischen Mittelwandlerchip (U4) umfasst; das phasengleiche Ende der zweiten Operationsverstärkereinheit (U2) ist mit einem gemeinsamen Knoten des zweiten Widerstands (R2) und der Blutäquivalenzimpedanzeinheit (RX) verbunden, und das außerphasige Ende der zweiten Operationsverstärkereinheit (U2) ist mit einem gemeinsamen Knoten des dritten Widerstands (R3) und des vierten Widerstands (R4) verbunden; der Ausgang der zweiten Operationsverstärkereinheit (U2) ist mit dem dritten Widerstand (R3) und dem Eingangsende des quadratischen Mittelkonverter-Chips (U4) verbunden, und das Eingangsende des quadratischen Mittelwandler-Chips (U4) ist verbunden mit einem gemeinsamen Knoten des Ausgangsendes der zweiten Operationsverstärkereinheit (U2) und des dritten Widerstands (R3); und das andere Ende des vierten Widerstands (R4) ist geerdet.

5. Detektionssystem nach Anspruch 4, wobei die Signalsammeleinheit (60) einen hochauflösenden Analog-Digital-Wandler (U6), eine Analog-Digital-Wandler-Ansteuerschaltung (U5) und eine präzise Spannungsreferenzeinheit umfasst; das Eingangsende der Analog-Digital-Wandler-Treiberschaltung (U5) ist mit dem Ausgangsende des quadratischen Mittelwandler-Chips (U4) verbunden, und das Ausgangsende der Analog-Digital-Wandler-Treiberschaltung (U5) ist mit dem Eingangsende des hochauflösenden Analog-Digital-Wandlers (U6) verbunden; der hochauflösende Analog-Digital-Wandler (U6) ist mit dem Ausgangsende der genauen Spannungsreferenzeinheit verbunden; und der hochauflösende Analog-Digital-Wandler (U6) ist mit der zentralen Verarbeitungseinheit (10) verbunden.

6. Detektionssystem nach Anspruch 5, wobei der monolithische Funktionsgenerator (U3) ein direkter digitaler Frequenzsynthesizer ist, der von der AD Company (Modell: AD9832) hergestellt wird; und der hochauflösende Analog-Digital-Wandler (U6) ist ein Delta-Sigma-Analog-Digital-Wandler.

7. Detektionssystem nach Anspruch 6, wobei zwei präzise Spannungsreferenzeinheiten vorgesehen sind, jeweils eine erste Präzisionsspannungsreferenzeinheit (REF1) und eine zweite Präzisionsspannungsreferenzeinheit (REF2); wobei die erste genaue Spannungsreferenzeinheit (REF1) mit der Spannungsreferenzschnittstelle des monolithischen Funktionsgenerators (U3) verbunden und konfiguriert ist, um eine präzise Spannungsreferenz für den monolithischen Funktionsgenerator (U3) bereitzustellen; und der hochauflösende Analog-Digital-Wandler (U6) ist mit dem Ausgangsende der zweiten Präzisionsspannungsreferenzeinheit (REF2) verbunden.

8. Betriebssteuerverfahren für ein Detektionssystem gemäß Anspruch 1, umfassend die folgenden Schritte:

Erzeugen einer Standard-Sinuswelle: Ausgeben einer Anweisung an die monolithische Anregungsquelleneinheit (70) durch die zentrale Verarbeitungseinheit (10), um die Standard-Sinuswelle zu erzeugen;
Sammeln eines Blutprobenimpedanzsignals, welches die Blutimpedanz anzeigt, mittels der Blutprobeneinheit (40);
Vervollständigen der Verstärkung (U2) des Blutprobenimpedanzsignals und der Umwandlung des Blutprobenimpedanzsignals auf einen effektiven Wert nach der Verstärkung mittels Messschaltungseinheit (50), um so ein Signal zur Verfügung zu stellen, welches den effektiven Wert der Blutimpedanz anzeigt;
Vervollständigen der Eintakt-zu-Differenzverstärkung (U5) und eine Analog-Digital-Umwandlung (U6) des Signals, welches den effektiven Wert der Blutimpedanz anzeigt, mittels der Signalsammeleinheit (60), um somit verarbeitete Daten zur Verfügung zu stellen, welche den effektiven Wert der Blutimpedanz anzeigen;
Berechnen des Blutimpedanzwerts gemäß den verarbeiteten Daten, die den effektiven Wert der Blutimpedanz anzeigen, mittels der zentralen Verarbeitungseinheit (10),
**dadurch gekennzeichnet, dass**
die Standard-Sinuswelle vom monolithischen Funktionsgenerator (U3) erzeugt wird, welcher in der Wellenformgeneratorschaltung (20) umfasst ist, welche in der Anregungsquelleneinheit (70) umfasst ist,
wobei Wellenformwandlerschaltung (30) ferner eine Gleichstromkomponente eines Ausgabesignals von der Wellenformgeneratorschaltung (20) isloliert und ein positives Sinussignal in eine positive und negative Halbzyklus-Sinuswelle wandelt;
wobei die genaue Spannungsreferenzeinheit ferner eine genaue Spannungsreferenz für den monolithischen Funktionsgenerator (U3) aufweist, wobei die Kristalloszillatoreinheit (X1) ein hochpräzises Taktsignal bereitzustellt und die Wellenformwandlerschaltung (30) die Gleichstromkomponente des Ausgabesignals von dem monolithischen Funktionsgenerator (U3) isoliert und das positive Sinussignal in die positive und negative Halbzyklus-Sinuswelle wandelt.

9. Verfahren nach Anspruch 8, ferner umfassend die folgenden Schritte:

B) Einstellen eines Standardwerts: Ausschalten eines Analogschalters, nicht Zugreifen auf eine Blutäquivalenzimpedanzeinheit zum Blut und Platzieren eines Standardwiderstands zum Skalieren in die Blutäquivalenzimpedanzeinheit;
C) Skalieren einer Schaltung: Setzen von M Skalierpunkten innerhalb eines typischen Impedanzmessbereichs von 1K bis 15K, wobei M größer oder gleich 2 ist, Starten der Schaltung einmal zum Skalieren jedes Mal, wenn ein Standardwiderstand in Schritt B zur Blutäquivalenzimpedanzeinheit gesetzt wird, und wiederholtes Ausführen von Schritt B und Schritt C für M-male; am Ende der Skalierung wird ein Codewert gespeichert, der durch ADC (Analog-Digital-Umwandlung) entsprechend jedem der Standardwiderstände in einem internen Speicher der Zentraleinheit erhalten wird, um eine Entsprechung zwischen dem durch ADC erhaltenen Codewert und dem Standardwiderstand zu erhalten;
D) Rücktest der Skalierung: Zugreifen auf einen Widerstand mit einem Widerstandswert von R zu der ursprünglichen Blutäquivalenzimpedanzeinheit Rx, Beurteilen, ob die Messabweichung einen voreingestellten Abweichungswert überschreitet; falls ja, Zurückkehren zu Schritt B; und falls nicht, Ausführen von Schritt E, wobei R von 1 KΩ bis 10 KΩ beträgt; und
E) Schaltungsselbstdetektion: Einschalten des Analogschalters, Zugreifen des den fünften Widerstands auf die Messschaltung, um den Widerstandswert des fünften Widerstands zu messen, Beurteilen, ob der Fehler des fünften Widerstands einen voreingestellten Wert überschreitet; falls dies der Fall ist, Ausgeben einer Aufforderung über die Anzeige einer Schaltungsabnormalität und automatisches Beenden der Messung und, falls nicht, Durchführen der Detektionsaufgabe durch das Detektionssystem.

**Revendications**

1. Système de détection destiné à l'amélioration de la précision de mesure d'hématocrite, comprenant une unité centrale de traitement (10), une unité de source d'excitation (70), une unité d'échantillon sanguin (40), une unité de circuit de mesure précise (50) et une unité de collecte de signal (60) ;

   dans lequel l'unité centrale de traitement (10) est connectée à une extrémité d'entrée de l'unité de source d'excitation (70), une extrémité de sortie de l'unité de source d'excitation (70) est connectée à une extrémité d'entrée de l'unité d'échantillon sanguin (40), dans lequel une extrémité de sortie de l'unité d'échantillon sanguin (40) est connectée à une extrémité d'entrée de l'unité de circuit de mesure précise (50), dans lequel une extrémité de sortie de l'unité de circuit de mesure précise (50) est connectée à l'unité de collecte de signal (60) et dans lequel l'unité centrale de traitement (10) est connectée à l'unité de collecte de signal (60) ;

   dans lequel l'unité centrale de traitement (10) est configurée pour produire en sortie une instruction à l'unité de source d'excitation (70) ; et l'unité de source d'excitation (70) est configurée pour générer une onde sinusoïdale standard selon l'instruction ;

   dans lequel l'unité d'échantillon sanguin (40) est configurée pour collecter un signal d'impédance de l'échantillon sanguin indique une impédance sanguine ;

   dans lequel l'unité de circuit de mesure précise (50) est configurée pour recevoir le signal d'impédance de l'échantillon sanguin provenant de l'unité d'échantillon sanguin (40) et terminer l'amplification du signal d'impédance de l'échantillon sanguin et la conversion du signal d'impédance de l'échantillon sanguin en une valeur effective après l'amplification, pour fournir ainsi un signal indiquant la valeur effective de l'impédance sanguine ;

   dans lequel l'unité de collecte de signal (60) est configurée pour recevoir le signal indiquant la valeur effective de l'impédance sanguine provenant de l'unité de circuit de mesure précise (50) et terminer l'amplification asymétrique-différentielle et la conversion analogique-numérique du signal indiquant la valeur effective de l'impédance sanguine, pour fournir ainsi des données traitées indiquant la valeur effective de l'impédance sanguine et l'unité de collecte de signal (60) est configurée pour produire en sortie les données traitées indiquant la valeur effective de l'impédance sanguine à l'unité centrale de traitement (10) ; et

   dans lequel l'unité centrale de traitement (10) est configurée en outre pour calculer une valeur d'impédance sanguine selon les données traitées indiquant la valeur effective de l'impédance sanguine et pour produire en sortie un ordre de commande à l'unité de collecte de signal (60) ;

   dans lequel l'unité de source d'excitation (70) comprend un circuit de générateur de forme d'onde (20) et un circuit du convertisseur de forme d'onde (30) connecté au circuit de générateur de forme d'onde (20) dans lequel le circuit générateur de forme d'onde (20) est configuré pour générer l'onde sinusoïdale standard et le circuit du convertisseur de forme d'onde (30) est configuré pour isoler une composante continue c.c. d'une sortie de signal provenant du circuit générateur de forme d'onde (20) et convertir un signal sinusoïdal positif en des ondes sinusoïdales demi-cycle positives et négatives ;

   **caractérisé en ce que** le circuit générateur de forme d'onde (20) comprend un générateur de fonction monolithique (U3) configuré pour générer l'onde sinusoïdale standard ;

   dans lequel le circuit générateur de forme d'onde (20) comprend en outre une unité d'oscillateur à cristal (X1) et une unité de référence de tension précise, l'unité de référence de tension précise étant connectée à une interface de référence de tension du générateur de fonction monolithique (U3) et configurée pour fournir une référence de tension précise au générateur de fonction monolithique (U3), le générateur de fonction monolithique (U3) étant connecté à l'unité d'oscillateur à cristal (X1), l'unité d'oscillateur à cristal (X1) étant configurée pour fournir un signal d'horloge de haute précision ; et l'extrémité d'entrée du circuit de générateur de forme d'onde (30) est connectée à l'extrémité de sortie du générateur de fonction monolithique (U3) et configurée pour isoler la composante continue c.c. d'une sortie de signal provenant du générateur de fonction monolithique (U3) et convertir le signal sinusoïdal positif en des ondes sinusoïdales demi-cycle positives et négatives.

2. Système de détection selon la revendication 1, dans lequel le circuit de convertisseur de forme d'onde (30) comprend un condensateur (C1), une première résistance (R1) et une première unité d'amplificateur opérationnel (U1) ; une des extrémités du condensateur (C1) est connectée à l'extrémité de sortie du générateur de fonctions monolithique (U3) et l'autre extrémité du condensateur (C1) est connectée à la résistance (R1) et l'extrémité d'entrée en phase de la première unité d'amplificateur opérationnel (U1) ; l'autre extrémité de la première résistance (R1) est à la masse ; l'extrémité en phase de la première unité d'amplificateur opérationnel (U1) est connectée à un noeud commun du condensateur (C1) et de la première résistance (R1) ; et une extrémité d'une deuxième résistance (R2) est connectée à l'extrémité de sortie de la première unité d'amplificateur opérationnel (U1).

3. Système de détection selon la revendication 2, dans lequel l'unité d'échantillon sanguin (40) est un circuit devant être testé ; l'unité d'échantillon sanguin (40) comprend une deuxième résistance (R2), une cinquième résistance (R5), un commutateur analogique (K1) et une unité d'impédance équivalente sanguine (RX) ; une extrémité de la deuxième résistance (R2) est connectée à l'extrémité de sortie de l'unité de source d'excitation (70) ; une extrémité de l'unité d'impédance équivalente sanguine (RX) est connectée à la deuxième résistance (R2) et l'autre extrémité de l'unité d'impédance équivalente sanguine (RX) est à la masse, une extrémité de la cinquième résistance (R5) est connectée à la deuxième résistance (R2) et l'autre extrémité de la cinquième résistance (R5) est connectée à une extrémité du commutateur analogique (K1) ; l'autre extrémité du commutateur analogique (K1) est à la masse ; et à la fois la deuxième résistance (R2) et la cinquième résistance (R5) sont des résistances standard.

4. Système de détection selon la revendication 3, dans lequel l'unité de circuit de mesure précise (50) comprend une deuxième unité d'amplificateur opérationnel (U2), une troisième résistance (R3), une quatrième résistance (R4) et une puce de convertisseur en racine carrée moyenne (U4) ; l'extrémité en phase de la deuxième unité d'amplificateur opérationnel (U2) est connectée à un noeud commun de la deuxième résistance (R2) et de l'unité d'impédance équivalente sanguine (RX) et l'extrémité hors phase de la deuxième unité d'amplificateur opérationnel (U2) est connectée à un noeud commun de la troisième résistance (R3) et de la quatrième résistance (R4) ; la sortie de la deuxième unité d'amplificateur opérationnel (U2) est connectée à la troisième résistance (R3) et l'extrémité d'entrée de la puce de convertisseur en racine carrée moyenne (U4) et l'extrémité d'entrée de la puce de convertisseur en racine carrée moyenne (U4) est connectée à un noeud commun de l'extrémité de sortie de la deuxième unité d'amplificateur opérationnel (U2) et de la troisième de la résistance (R3) ; et l'autre extrémité de la quatrième la résistance (R4) est à la masse.

5. Système de détection selon la revendication 4, dans lequel l'unité de collecte de signal (60) comprend un convertisseur analogique-numérique à haute résolution (U6), un circuit de commande du convertisseur analogique-numérique (U5) et une unité de référence de tension précise ; l'extrémité d'entrée du circuit de commande du convertisseur analogique-numérique (U5) est connectée à l'extrémité de sortie de la puce de convertisseur en racine carrée moyenne (U4) et l'extrémité de sortie du circuit de commande du convertisseur analogique-numérique (U5) est connectée à l'extrémité d'entrée du convertisseur analogique-numérique à haute résolution (U6) ; le convertisseur analogique-numérique à haute résolution (U6) est connecté à l'extrémité de sortie de l'unité de référence de tension précise et le convertisseur analogique-numérique à haute résolution (U6) est connecté à l'unité centrale de traitement (10).

6. Système de détection selon la revendication 5, dans lequel le générateur de fonction monolithique (U3) est un synthétiseur de fréquence numérique directe qui est produit par la compagnie AD (modèle : AD9832) ; et le convertisseur analogique-numérique à haute résolution (U6) est un convertisseur analogique-numérique delta-sigma.

7. Système de détection selon la revendication 6, dans lequel il existe deux unités de référence de tension précise, respectivement une première unité de référence de tension précise (REF1) et une deuxième unité de référence de tension précise (REF2) ; la première unité de référence de tension précise (REF1) est connectée à l'interface de référence de tension du générateur de fonctions monolithique (U3) et configurée pour fournir une référence de tension précise au générateur de fonction monolithique (U3) ; et le convertisseur analogique-numérique à haute résolution (U6) est connecté à l'extrémité de sortie de la deuxième unité de référence de tension précise (REF2).

8. Procédé d'utilisation du système de détection selon la revendication 1, comprenant les étapes suivantes :

génération d'une onde sinusoïdale standard, qui comprend : produire en sortie, par l'unité centrale de traitement (10), une instruction à l'unité de source d'excitation (70) pour générer l'onde sinusoïdale standard ;
collecte, par l'unité d'échantillon sanguin (40), d'un signal d'impédance de l'échantillon sanguin indiquant une impédance sanguine ;
terminaison, par l'unité de circuit de mesure précise (50), de l'amplification du signal d'impédance de l'échantillon sanguin et de la conversion du signal d'impédance de l'échantillon sanguin en une valeur effective après l'amplification, pour fournir ainsi un signal indiquant la valeur effective de l'impédance sanguine ;
terminaison, par l'unité de collecte de signal (60), de l'amplification asymétrique-différentielle et de la conversion analogique-numérique du signal indiquant la valeur effective de l'impédance sanguine, pour fournir ainsi des données traitées indiquant la valeur effective de l'impédance sanguine ;
calcul, par l'unité centrale de traitement (10), d'une valeur d'impédance sanguine selon les données traitées indiquant la valeur effective de l'impédance sanguine ;
**caractérisée en ce que** l'onde sinusoïdale standard est générée par le générateur de fonction monolithique (U3) compris dans le circuit générateur de forme d'onde (20) compris dans l'unité de source d'excitation (70) ;
dans lequel le circuit de convertisseur de forme d'onde (30) isole en outre une composante continue c.c. d'une sortie de signal provenant du circuit générateur de forme d'onde (20) et convertit un signal sinusoïdal positif en des ondes sinusoïdales demi-cycle positives et négatives ;

dans lequel l'unité de référence de tension précise fournit en outre une référence de tension précise au générateur de fonction monolithique (U3), l'unité d'oscillateur à cristal (X1) fournit un signal d'horloge de haute précision et le circuit du convertisseur de forme d'onde (30) isole la composante continue c.c. d'une sortie de signal provenant du générateur de fonction monolithique (U3) et convertit le signal sinusoïdal positif en des ondes sinusoïdales demi-cycle positives et négatives.

9. Procédé selon la revendication 8, comprenant en outre les étapes suivantes :

B) définition d'une valeur standard : désactiver un commutateur analogique, ne pas accéder à une unité d'impédance équivalente sanguine pour le sang et placer une résistance standard destinée à mettre à l'échelle l'unité d'impédance équivalente sanguine ;

C) mise à l'échelle d'un circuit : définir M points de mise à l'échelle à l'intérieur d'une plage de mesure d'impédance typique, allant de 1K à 15K, dans laquelle M est supérieur ou égal à 2, démarrer le circuit une fois pour la mise à l'échelle chaque fois qu'une résistance standard est placée sur l'unité d'impédance équivalente sanguine dans l'étape B et exécuter de manière répétée M fois l'étape B et l'étape C ; à la fin de la mise à l'échelle, enregistrer une valeur de code obtenue par ADC (conversion analogique-numérique) correspondant à chacune des résistances standard dans une mémoire interne de l'unité centrale de traitement pour obtenir une correspondance entre la valeur de code obtenue par ADC et la résistance standard;

D) contrôle a posteriori de la mise à l'échelle : accéder à une résistance présentant une résistance R sur l'unité d'impédance équivalente sanguine d'origine Rx, juger si oui ou non la déviation de mesure dépasse une valeur de déviation prédéfinie, si oui, retourner à l'étape B ; sinon, exécuter l'étape E, dans laquelle R va de 1 K$\Omega$ à 10 K$\Omega$; et

E) autodétection du circuit : activer le commutateur analogique, accéder à la cinquième résistance au circuit de mesure pour mesurer la résistance de la cinquième résistance, juger si oui ou non l'erreur de la cinquième résistance dépasse une valeur prédéfinie, si oui, donner une indication rapide d'anomalie du circuit et mettre fin automatiquement à la mesure et sinon, effectuer la tâche de détection par le système de détection.

Fig. 1

Fig. 2

EP 2 955 501 B1

EP 2 955 501 B1

Fig. 3

Fig. 4

EP 2 955 501 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102006014825 A1

- CN 101889863 A